# EUROPEAN PATENT SPECIFICATION

(11) **EP 4 334 275 B1**
(45) Date of publication and mention of the grant of the patent: **21.01.2026**
(21) Application number: 22725114.7
(22) Date of filing: 05.05.2022
(51) Int. Cl.: C07F 7/18, C07C 41/48, C07C 43/313

(54) **HALOCYCLOPROPANATION PROCESSES**
HALOCYCLOPROPANIERUNGSVERFAHREN
PROCÉDÉS D'HALOCYCLOPROPANATION

(30) Priority: 07.05.2021 US 202163185399 P
(43) Date of publication of application: 13.03.2024
(73) Proprietor: Corteva Agriscience LLC, Indianapolis, IN 46268 (US)
(72) Inventor: BARTON, Thomas J., Indianapolis, Indiana 46220 (US); CABRERA VENTURA, Pablo Jose, Zionsville, Indiana 46077 (US); GOOD, Steffen N., Indianapolis, Indiana 46268 (US); LI, Fangzheng, Indianapolis, Indiana 46268 (US); ROSENTHAL, Tay, Indianapolis, Indiana 46268 (US); SCHUITMAN, Abraham D., Indianapolis, Indiana 46268 (US); VERMEULEN, Nicolaas A., Chicago, Illinois 60640 (US)
(74) Representative: f & e patent
(86) International application number: PCT/US2022/027768
(87) International publication number: WO 2022/235863

(56) References cited:
- WO-A1-2016/168059
- WO-A1-2021/216629

## Description

### CROSS REFERENCE TO RELATED APPLICATIONS

This application claims the benefit of, and priority from, U.S. provisional application serial number 63/185,399, which was filed 07 MAY 2021.

### FIELD OF THIS DISCLOSURE

This disclosure relates to halocyclopropanation processes useful in forming molecules having pesticidal utility against pests in Phyla Arthropoda, Mollusca, and Nematoda.

### BACKGROUND OF THIS DISCLOSURE

Halocyclopropanation processes have been disclosed in applications WO/2016/168056; WO/2016/168058; WO/2016/168059; WO/2018/071320; and WO/2018/071327. WO 2016/168059A1 discloses the general preparation of phenylcyclopropyl acetals, where chloroform and a base is used for generating dichlorocarbene.

### DEFINITIONS USED IN THIS DISCLOSURE

The examples given in these definitions are not exhaustive. It is understood that a substituent should comply with chemical bonding rules and steric compatibility constraints in relation to the particular molecule to which it is attached. These definitions are only to be used for the purposes of this disclosure.

The term **"alkoxy"** means an alkyl further consisting of a carbon-oxygen single bond, for example, methoxy, ethoxy, propoxy, isopropoxy, butoxy, sec-butoxy, isobutoxy, and tert-butoxy.

The term **"alkyl"** means an acyclic, saturated, branched, or unbranched, substituent consisting of carbon and hydrogen, for example, methyl, ethyl, propyl, isopropyl, butyl, sec-butyl, isobutyl, and *tert-*butyl.

The term **"aryl"** means a cyclic, aromatic substituent consisting of hydrogen and carbon, for example, phenyl, naphthyl, and biphenyl.

The term **"halo"** means fluoro, chloro, bromo, and iodo.

The term **"haloalkoxy"** means an alkoxy further consisting of, from one to the maximum possible number of identical or different, halos, for example, fluoromethoxy, trifluoromethoxy, 2,2-difluoropropoxy, chloromethoxy, trichloromethoxy, 1,1,2,2-tetrafluoroethoxy, and pentafluoroethoxy.

The term **"haloalkyl"** means an alkyl further consisting of, from one to the maximum possible number of, identical or different, halos, for example, fluoromethyl, trifluoromethyl, 2,2-difluoropropyl, chloromethyl, trichloromethyl, and 1,1,2,2-tetrafluoroethyl.

### DETAILED DESCRIPTION OF THIS DISCLOSURE

Scheme One is shown below.

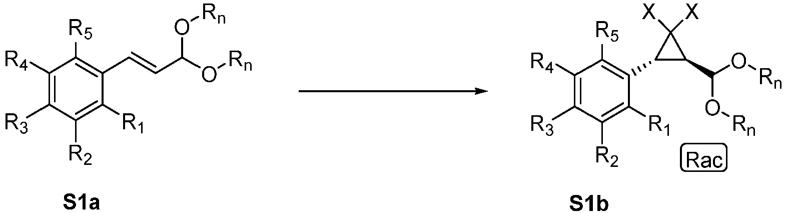

In Scheme One:
**(a) R₁, R₂, R₃, R₄,** and **R₅** are each independently H, F, Cl, Br, I, CN, NH₂, NO₂, (C₁-C₄)alkyl, (C₁-C₄)alkoxy, (C₁-C₄)haloalkyl, or (C₁-C₄)haloalkoxy;
**(b)** each **Rₙ** is independently a (C₁-C₄)alkyl, or both Rₙ substituents together form a (C₂-C₆)alkyl link between the two oxygen atoms; and
**(c)** each **X** is independently Cl.

In one embodiment, **R₁, R₂, R₃, R₄,** and **R₅** are each independently H, F, Cl, Br, I, CN, NH₂, NO₂, (C₁-C₄)alkyl, (C₁-C₄)alkoxy, (C₁-C₄)haloalkyl, or (C₁-C₄)haloalkoxy, with the proviso that at least one of **R₂, R₃,** and **R₄** is not H.

In another embodiment each **X** is independently **Cl.**

In another embodiment of **S1a: R₂** and **R₄** are CF₃; **R₁, R₃,** and **R₅** are H; and each **Rₙ** is C₂H₅. This molecule is **(*E*)-1-(3,3-diethoxyprop-1-en-1-yl)-3,5-bis(trifluoromethyl)benzene,** hereafter referred to as (**"S1a-1"**)**.**

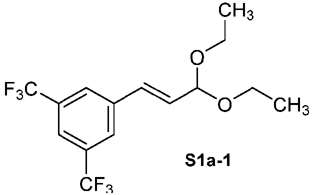

The reaction in Scheme One is conducted in the presence of a halocyclopropanating reagent that reacts with **S1a** to produce **S1b.** In other words, functionally the halocyclopropanating reagent acts as a source of dihalocarbene. Examples of such halocyclopropanating reagents are carbon tetrachloride (CCl₄), tetrachloroethylene (Cl₂C-CCl₂), chloroform (CHCl₃), salts of trichloroacetic acid (metals including but not limited to lithium, sodium, potassium, and quaternary ammonium cations), alkyl esters of trichloroacetate, hexafluoroacetone ((F₃C)₂CO), hexachloroacetone ((Cl₃C)₂CO, also known as 1,1,1,3,3,3-hexachloropropan-2-one), and hexabromoacetone ((Br₃C)₂CO). In general, while the amount that may be used depends on a particular reagent, a range from about 0.5 moles to about 100 moles of a reagent may be used per mole of **S1a.** For example, when chloroform is the reagent, a range from about 5 moles to about 100 moles may be used; however, in some cases a range from about 30 moles to about 60 moles may be used. As another example, when hexachloroacetone is the reagent, a range from about 0.5 moles to about 100 moles may be used; however, in some cases, a range from about 1 mole to about 5 moles may be used. Mixtures of halocyclopropanating agents may also be used.

The reaction in Scheme One is conducted in the presence of lithium salts of tertiary alkoxides. For example, lithium tert-butoxide (LiOC(CH₃)₃, also known as lithium 2-methylpropan-2-olate) and lithium 2-methyl-2-butoxide (also known as lithium *tert*-amoxide or lithium tert-amylate) may be used. In general, an amount in the range from about 1 mole to about 20 moles per mole of S1a may be used; from about 1 mole to about 15 moles may also be used. Optionally, mixtures of such lithium salts may be used.

The reaction in Scheme One may be conducted in the presence of a co-solvent. Such co-solvents, for example, may be selected from one or more of the following: heptane, hexane, toluene, diglyme, tetrahydrofuran ("THF"), dimethyl carbonate, dimethoxyethane, ethylene glycol methyl ether, tert-butanol ("t-BuOH"), nitrobenzene, chlorobenzene, methyl *tert*-butyl ether ("MTBE"), 2-methyldecane, and anisole (also known as methoxybenzene). Optionally, mixtures of such co-solvents, for example mixtures of C₉-C₁₂ isoalkanes, may be used.

Optionally, the reaction in Scheme One may be conducted in the presence of a phase-transfer catalyst. Such phase-transfer catalysts, for example, benzyltributylammonium chloride, benzytriethylammonium choride, tetrabutylammonium bromide, tetrabutylammonium hexachlorophosphate, *N*-benzylcinchonidium chloride, (-)-*N*-dodecyl-*N*-methylephedrinium bromide, Aliquat^{®} 336 (trioctylmethylammonium chloride). Optionally, mixtures of phase-transfer catalysts may be used.

The reaction in Scheme One may be conducted at ambient temperatures and ambient pressures. However, higher and lower temperatures and pressures may be used. Currently, temperatures from about 0 °C to about 100 °C may be used. However, depending on the reagent or reagents used, other ranges may be used. For example, when chloroform is the reagent, a range from about 0 °C to about 60 °C may be used; in some situations, a range from about 20 °C to about 50 °C may be used; and in other situations, a range from about 25 °C to about 40 °C may be used. As another example, when hexachloroacetone is the reagent, a range from about 0 °C to about 40 °C may be used; however, in some cases a range from about 0 °C to about 25 °C may be used. A pressure from about 10 kilopascal (kPa) to about 1000 kPa may be used, and in some situations a pressure from about 50 kPa to about 150 kPa may be used.

Scheme Two is shown below.

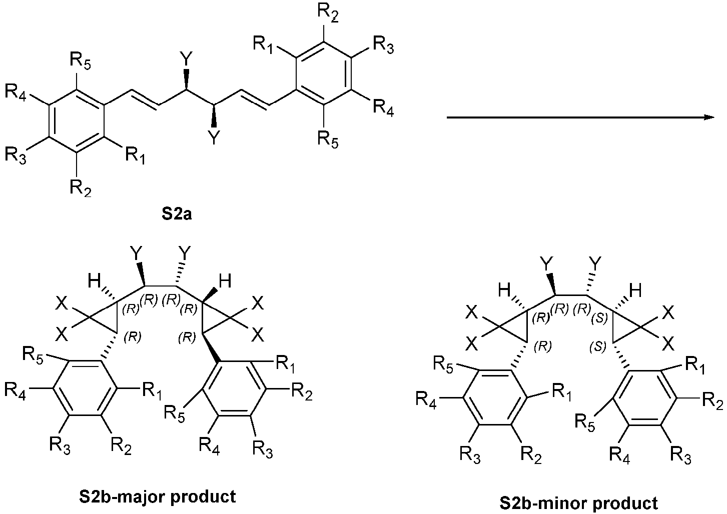

In Scheme Two:
**(a) R₁, R₂, R₃, R₄,** and **R₅** are each independently H, F, Cl, Br, I, CN, NH₂, NO₂, (C₁-C₄)alkyl, (C₁-C₄)alkoxy, (C₁-C₄)haloalkyl, or (C₁-C₄)haloalkoxy;
**(b)** each **Y** is independently a OH, OSi((C₁-C₄)alkyl)₃, OSi((C₁-C₄)alkyl)₂((C₁-C₄)alkyl-aryl), OSi((C₁-C₄)alkyl)₂(aryl), OSi((C₁-C₄)alkyl)(aryl)₂, OC(O)(C₁-C₄)alkyl, OC(O)O(C₁-C₄)alkyl, OC(O)NH(C₁-C₄)alkyl, O(C₁-C₄)alkyl-aryl, O-aryl, tetrahydropyranyl, or 1,3-dioxolanyl; and
**(c)** each **X** is independently Cl.

In one embodiment, **R₁, R₂, R₃, R₄,** and **R₅** are each independently H, F, Cl, Br, I, CN, NH₂, NO₂, (C₁-C₄)alkyl, (C₁-C₄)alkoxy, (C₁-C₄)haloalkyl, or (C₁-C₄)haloalkoxy, with the proviso that at least one of **R₂, R₃,** and **R₄** is not H.

In another embodiment **Y** is independently OSi(CH₃)₃, OSi(CH₂CH₃)₃, OSi(CH(CH₃)₂)₃, OSi(CH₃)₂phenyl, OBenzyl, O(C(O)NH-*tert*-butyl), OSi(CH₃)₂-(*tert*-butyl).

In another embodiment each **X** is independently **Cl.**

In another embodiment of **S2a: R₂** and **R₄** are CF₃; **R₁, R₃,** and **R₅** are H; and Y is OSi(CH₃)₂(*tert-*butyl). This molecule is **(5*R*,6*R*)-5,6-bis((*E*)-3,5-bis(trifluoromethyl)styryl)-2,2,3,3,8,8,9,9-octamethyl-4,7-dioxa-3,8-disiladecane,** hereafter referred to as (**"S2a-1"**)**.**

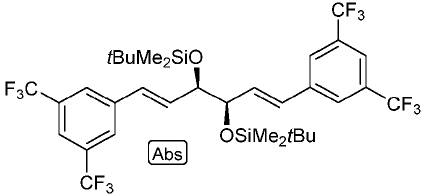

The reaction in Scheme Two is conducted in the presence of a halocyclopropanating reagent that converts **S2a to S2b-major product.** In other words, functionally the halocyclopropanating reagent acts as a source of dihalocarbene. Examples of such halocyclopropanating reagents are carbon tetrachloride (CCl₄), tetrachloroethylene (Cl₂C-CCl₂), chloroform (CHCl₃), salts of trichloroacetic acid (metals including but not limited to lithium, sodium, potassium, and quaternary ammonium cations), alkyl esters of trichloroacetate, hexafluoroacetone ((F₃C)₂CO), hexachloroacetone ((Cl₃C)₂CO, also known as 1,1,1,3,3,3-hexachloropropan-2-one), and hexabromoacetone ((Br₃C)₂CO). In general, while the amount that may be used depends on a particular reagent, a range from about 0.5 moles to about 100 moles of a reagent may be used per mole of **S2a.** For example, when chloroform is the reagent, a range from about 5 moles to about 100 moles may be used; however, in some cases a range from about 30 moles to about 60 moles may be used. As another example, when hexachloroacetone is the reagent, a range from about 0.5 moles to about 100 moles may be used; however, in some cases, a range from about 1 mole to about 5 moles may be used. Mixtures of halocyclopropanating agents may also be used.

The reaction in Scheme Two is conducted in the presence of lithium salts of tertiary alkoxides. For example, lithium *tert*-butoxide (LiOC(CH₃)₃, also known as lithium 2-methylpropan-2-olate) and lithium 2-methyl-2-butoxide (also known as lithium tert-amoxide or lithium *tert*-amylate) may be used. In general, an amount in the range from about 1 mole to about 20 moles per mole of **S2a** may be used; from about 1 mole to 15 moles may also be used. Optionally, mixtures of such lithium salts may be used.

The reaction in Scheme Two may be conducted in the presence of a co-solvent. Such co-solvents, for example, may be selected from one or more of the following: heptane, hexane, toluene, diglyme, tetrahydrofuran ("THF"), dimethyl carbonate, dimethoxyethane, ethylene glycol methyl ether, tert-butanol ("t-BuOH"), nitrobenzene, chlorobenzene, methyl tert-butyl ether ("MTBE"), 2-rnethyldecane, and anisole (also known as methoxybenzene). Optionally, mixtures of such co-solvents, for example mixtures of C₉-C₁₂ isoalkanes, may be used.

Optionally, the reaction in Scheme Two may be conducted in the presence of a phase-transfer catalyst. Such phase-transfer catalysts, for example, benzyltributylammonium chloride, benzytriethylammonium choride, tetrabutylammonium bromide, tetrabutylammonium hexachlorophosphate. *N*-benzylcinchonidium chloride, (-)-*N*-dodecyl-*N*-methylephedrinium bromide, Aliquat^{®} 336 (trioctylmethylammonium chloride). Optionally, mixtures of phase-transfer catalysts may be used.

The reaction in Scheme Two may be conducted at ambient temperatures and ambient pressures. However, higher and lower temperatures and pressures may be used. Currently, temperatures from about 0 °C to about 100 °C may be used. However, depending on the reagent or reagents used, other ranges may be used. For example, when chloroform is the reagent, a range from about 0 °C to about 60 °C may be used; in some situations, a range from about 20 °C to about 50 °C may be used; and in other situations, a range from about 25 °C to about 40 °C may be used. As another example, when hexachloroacetone is the reagent, a range from about 0 °C to about 40 °C may be used; however, in some cases a range from about 0 °C to about 25 °C may be used. A pressure from about 10 kilopascal (kPa) to about 1000 kPa may be used, and in some situations a pressure from about 50 kPa to about 150 kPa may be used.

### EXAMPLES

These examples are for illustration purposes and are not to be construed as limiting this disclosure to only the embodiments disclosed in these examples.

Starting materials, reagents, and solvents that were obtained from commercial sources were used without further purification. Anhydrous solvents were purchased as Sure/Seal^{™} from Aldrich and were used as received. Bases and other reagents were stored in a nitrogen (N₂)-filled glovebox, unless otherwise stated. Examples using "ambient temperature" were conducted in climate controlled laboratories with temperatures ranging from about 20 °C to about 24 °C. Molecules are given their known names, named according to naming programs within ChemDraw. If such a program is unable to name a molecule, such molecule is named using conventional IUPAC naming rules. ¹H NMR spectral data are in ppm (δ) and were recorded at 400 and 500 MHz; ¹³C NMR spectral data are in ppm (δ) and were recorded at 75, 100, or 150 MHz; and ¹⁹F NMR spectral data are in ppm (δ) and were recorded at 471 MHz, unless otherwise stated.

### Example 1a: Synthesis of trans-rac-1-(2,2-dichloro-3-(diethoxymethyl)cyclopropyl)-3,5-bis(trifluoromethyl)benzene

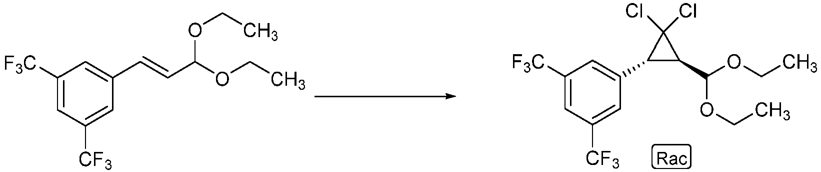

In a 250 milliliter (mL) three-neck round bottom flask equipped with magnetic stir bar and temperature probe, (*E*)-1-(3,3-diethoxyprop-1-en-1-yl)-3,5-bis(trifluoromethyl)benzene (5 grams (g), 14.61 millimoles (mmol)) was dissolved in chloroform (47.1 mL, 584 mmol). Lithium 2-methylpropan-2-olate (11.69 g, 146 mmol) was added to the solution in one portion. The mixture was heated to 40 °C and stirred vigorously for 24 hours. After cooling to ambient temperature, the reaction mixture was washed with water (50 mL) twice and then with brine. The organic layer was separated, dried over anhydrous sodium sulfate, filtered, and concentrated under vacuum by rotary evaporator to obtain the resulting oil containing the desired product (72% conversion to product). Starting material and product were inseparable by silica gel column chromatography (hexanes-ethyl acetate eluent): ¹H NMR (400 MHz, CDCl₃) δ 7.83 (s, 1H), 7.71 (s, 2H), 4.64 (d, *J* = 6.1 Hz, 1H), 3.82 - 3.55 (m, 4H), 2.94 (d, *J* = 8.4 Hz, 1H), 2.35 (dd, *J* = 8.5, 6.1 Hz, 1H), 1.32 (t, *J* = 7.0 Hz, 3H), 1.21 (t, *J* = 7.1 Hz, 3H); ¹⁹F NMR (471 MHz, CDCl₃) δ -62.87.

### Example1b: Synthesis of trans-rac-(2,2-dichloro-3-(diethoxymethyl)cyclopropyl)benzene

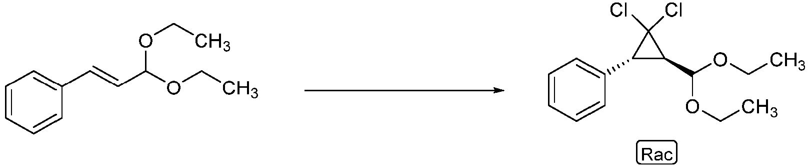

In a vial, (*E*)-(3,3-diethoxyprop-1-en-1-yl)benzene (200 milligrams (mg), 0.97 mmol) was dissolved in chloroform (3.128 mL, 38.8 mmol). The solution was added to another vial containing lithium tert-butoxide (776 mg, 9.70 mmol, 10 equivalents relative to starting material) weighed out in a glovebox. The reaction was heated to 40 °C and stirred vigorously for 24 hours. After cooling to ambient temperature, the reaction mixture was washed with water (2 mL) twice. The organic layer was separated, dried over anhydrous sodium sulfate, filtered, and concentrated under vacuum by rotary evaporator. The resulting oil was analyzed by ¹H NMR spectroscopy to obtain ¹H-NMR yield using 1,4-dinitrobenzene as an internal standard (82% yield): ¹H NMR (500 MHz, CDCl₃) δ 7.40 - 7.26 (m, 5H), 4.61 (d, *J* = 6.5 Hz, 1H), 3.90 - 3.53 (m, 4H), 2.84 (d, *J* = 8.6 Hz, 1H), 2.31 (dd, *J* = 8.6, 6.5 Hz, 1H), 1.31 (t, *J* = 7.1 Hz, 3H), 1.19 (t, *J* = 7.0 Hz, 3H).

### Example1c: Synthesis of trans-rac-1,3-dichloro-5-(2,2-dichloro-3-diethoxymethyl)cyclopropyl)benzene

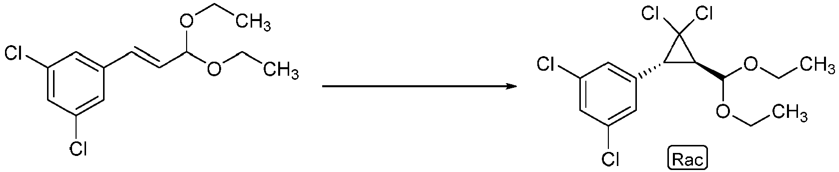

In a vial, (*E*)-1,3-dichloro-5-(3,3-diethoxyprop-1-en-1-yl)benzene (200 mg, 0.727 mmol, 97 weight percent (wt %)) was dissolved in chloroform (2.345 mL, 29.1 mmol). The solution was added to another vial containing lithium *tert*-butoxide (582 mg, 7.27 mmol, 10 equivalents relative to starting material) weighed out in a glovebox. The reaction mixture was heated to 40 °C and stirred vigorously for 24 hours. After cooling to ambient temperature, the reaction mixture was washed with water (2 mL) twice. The organic layer was separated, dried over anhydrous sodium sulfate, filtered, and concentrated under vacuum by rotary evaporator. The resulting oil was analyzed by ¹H NMR spectroscopy to obtain ¹H-NMR yield using 1,4-dinitrobenzene as an internal standard (71% yield): ¹H NMR (500 MHz, CDCl₃) δ 7.31 (t, *J* = 1.9 Hz, 1H), 7.16 - 7.14 (m, 2H), 4.59 (d, *J* = 6.1 Hz, 1H), 3.80 - 3.47 (m, 4H), 2.77 (d, *J* = 8.5 Hz, 1H), 2.25 (dd, J = 8.5, 6.2 Hz, 1H), 1.30 (t, *J* = 7.1 Hz, 3H), 1.20 (t, *J* = 7.0 Hz, 3H).

### Example 2a: Synthesis of trans-rac-1-(2,2-dichloro-3-(diethoxymethyl)cyclopropyl)-3,5-bis(trifluoromethyl)benzene

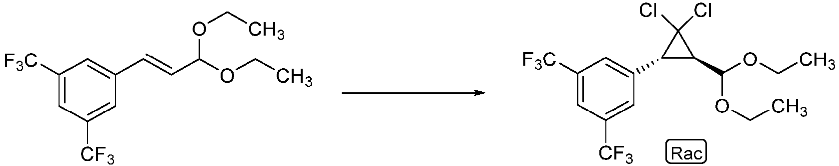

In a single-neck 50-mL round bottom flask equipped with a nitrogen inlet and magnetic stir bar was added (*E*)-1-(3,3-diethoxyprop-1-en-1-yl)-3,5-bis(trifluoromethyl)benzene (5.00 g, 14.61 mmol, 1 equivalent). Hexachloroacetone (19.34 g, 13.43 mL, 73.0 mmol, 5 equivalents) was added to the flask. Lithium *tert-*butoxide (3.51 g, 43.8 mmol, 3 equivalents) was added in one portion at 23 °C. The reaction mixture was stirred at 23 °C under a nitrogen atmosphere for 24 hours. Heptane (20 mL) was added. The mixture was washed with water (10 mL) three times. The heptane layer was concentrated. Purification by flash column chromatography on silica gel (eluent: 0 - 50% ethyl acetate in hexanes) afforded the title compound as a yellow solid (5.28 g, 85% yield): ¹H NMR (500 MHz, CDCl₃) δ 7.83 (s, 1H), 7.71 (d, *J* = 1.6 Hz, 2H), 4.64 (d, *J* = 6.1 Hz, 1H), 3.81 - 3.67 (m, 3H), 3.66 - 3.57 (m, 1H), 2.93 (d, *J* = 8.5 Hz, 1H), 2.35 (dd, *J* = 8.5, 6.0 Hz, 1H), 1.32 (t, *J* = 7.1 Hz, 3H), 1.21 (t, *J* = 7.1 Hz, 3H); ¹⁹F NMR (471 MHz, CDCl₃) δ -62.89.

### Example 2b: Synthesis of rac-(2,2-dichloro-3-(diethoxymethyl)cyclopropyl)benzene

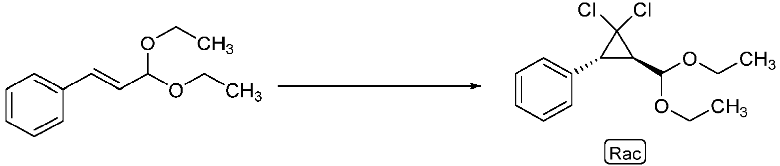

In a vial, (*E*)-(3,3-diethoxyprop-1-en-1-yl)benzene (0.200 g, 0.970 mmol) was dissolved in hexachloroacetone (0.738 mL, 4.85 mmol, 5 equivalents relative to starting material). The solution was added to another vial containing lithium tert-butoxide (0.233 g, 2.91 mmol, 3 equivalents relative to starting material) weighed out in a glove box. The reaction mixture was stirred vigorously for 24 hours at 23 °C. 1,3,5-Trimethoxybenzene was added to the reaction mixture and used as internal standard to calculate yield by ¹H NMR spectroscopy (91% yield).

### Example 2c: Synthesis of trans-rac-1-(2,2-dichloro-3-(diethoxymethyl)cyclopropyl)-3,5-bis(trifluoromethyl)benzene

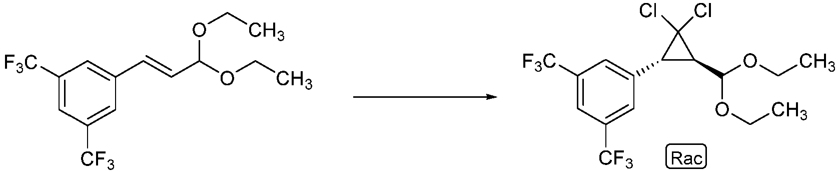

In a vial, (*E*)-1,3-dichloro-5-(3,3-diethoxyprop-1-en-1-yl)benzene (200 mg, 0.727 mmol, 97 wt %) was dissolved in hexachloroacetone (0.553 mL, 3.63 mmol). The solution was added to another vial containing lithium tert-butoxide (175 mg, 2.18 mmol, 3 equivalents relative to starting material) weighed out in a glovebox. The reaction mixture was stirred vigorously for 24 hours at 23 °C. 1,3,5-Trimethoxybenzene was added to the reaction mixture and used as internal standard to calculate yield by ¹H NMR spectroscopy (98% yield): ¹H NMR (500 MHz, CDCl₃) δ 7.31 (t, *J* = 1.9 Hz, 1H), 7.16 - 7.14 (m, 2H), 4.59 (d, *J* = 6.2 Hz, 1H), 3.81 - 3.56 (m, 4H), 2.77 (d, *J* = 8.5 Hz, 1H), 2.25 (dd, *J* = 8.5, 6.2 Hz, 1H), 1.30 (t, *J* = 7.0 Hz, 3H), 1.20 (t, *J* = 7.1 Hz, 3H).

### Example 2d: Synthesis of trans-rac-1-(2,2-dichloro-3-(diisopropoxymethyl)cyclopropyl)-3,5-bis(trifluoromethyl)benzene

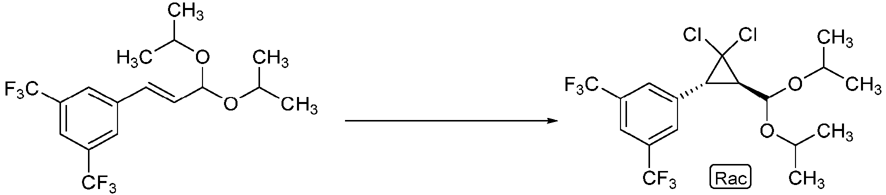

In a vial, (*E*)-1-(3,3-diisopropoxyprop-1-en-1-yl)-3,5-bis(trifluoromethyl)benzene (200 mg, 0.540 mmol, 90 wt %) was dissolved in hexachloroacetone (0.411 mL, 2.70 mmol). The solution was added to another vial containing lithium tert-butoxide (130 mg, 1.62 mmol, 3 equivalents relative to starting material) weighed out in a glovebox. The reaction was stirred vigorously for 24 hours at 23 °C. 1,3,5-Trimethoxybenzene was added to the reaction mixture and used as internal standard to calculate yield by ¹H NMR spectroscopy (71% yield): ¹H NMR (500 MHz, CDCl₃) δ 7.83 (s, 1H), 7.73 (s, 2H), 4.68 (d, *J* = 6.5 Hz, 1H), 4.12 - 4.07 (m, 1H), 4.00 - 3.94 (m, 1H), 2.85 (d, *J* = 8.5 Hz, 1H), 2.34 (dd, *J* = 8.5, 6.4 Hz, 1H), 1.31 (d, *J* = 6.2 Hz, 3H), 1.28 (d, *J* = 4.9 Hz, 3H), 1.19 (d, *J* = 6.4 Hz, 3H), 1.14 (d, *J* = 6.1 Hz, 3H); ¹⁹F NMR (471 MHz, CDCl₃) δ - 62.89.

### Example 3a: Comparison of bases used in reaction to form trans-rac-1-(2,2-dichloro-3-(diethoxymethyl)cyclopropyl)-3,5-bis(trifluoromethyl)benzene from (E)-1-(3,3-diethoxyprop-1-en-1-yl)-3,5-bis(trifluoromethyl)benzene

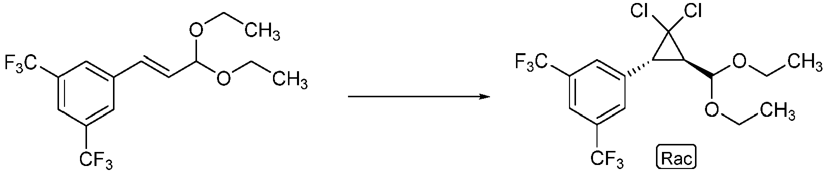

The following general procedure was used. Into a scintillation vial inside the glovebox, the base (5.84 mmol, 10 equivalents) was weighed. In a separate vial, (*E*)-1-(3,3-diethoxyprop-1-en-1-yl)-3,5-bis(trifluoromethyl)benzene (0.20 g, 0.584 mmol, 1 equivalent) and chloroform (1.885 mL, 23.37 mmol, 40 equivalents) were mixed and then added to the base via syringe at 23 °C. The mixture was stirred for 24 hours at 23 °C. 1,4-Dinitrobenzene was added to the reaction mixture and used as internal standard to calculate yield by ¹H NMR spectroscopy. The results are reported in the table below.

| **TABLE 3a** | | |
|---|---|---|
| **Base (molar equivalents with respect to starting material)** | **Starting Material conversion** | **Product ¹H-NMR yield** |
| 2-Methylbutan-2-olate (10 equivalents) | 70% | 71% |
| Lithium *tert*-butoxide (10 equivalents) | 74% | 78% |
| Lithium isopropoxide (10 equivalents) | 0.55% | 0.57% |
| Lithium ethoxide (10 equivalents) | 0.35% | 0.48% |
| Lithium methoxide (10 equivalents) | Not determined | Not detected |

### Example 3b: Comparison of bases used in reaction to form trans-rac-1-(2,2-dichloro-3-(diethoxymethyl)cyclopropyl)-3,5-bis(trifluoromethyl)benzene from (E)-1-(3,3-diethoxyprop-1-en-1-yl)-3,5-bis(trifluoromethyl)benzene

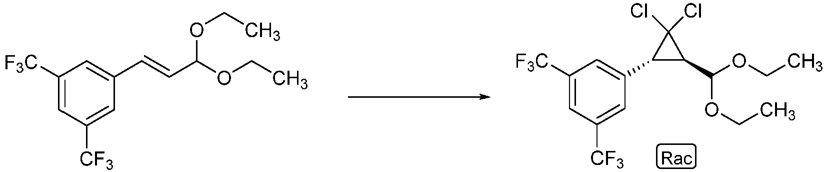

Into a scintillation vial inside the glovebox, the base (1.753, 3 equivalents) was weighed. In a separate vial, (*E*)-1-(3,3-diethoxyprop-1-en-1-yl)-3,5-bis(trifluoromethyl)benzene (0.20 g, 0.584 mmol, 1 equivalent) and hexachloroacetone (0.445 mL, 2.92 mmol, 5 equivalents) were mixed and then added to the solid base via syringe at 23 °C. The mixture was stirred for 24 hours at 23 °C. 1,3,5-Trimethoxybenzene was added to the reaction mixture and used as internal standard to calculate yield by ¹H NMR spectroscopy. The results are reported in the table below.

| **TABLE 3b** | | |
|---|---|---|
| **Base (molar equivalents with respect to starting material)** | **Starting Material conversion** | **Product ¹H-NMR yield** |
| Lithium *tert*-amylate (3 equivalents) | 80% | 63% |
| Lithium *tert*-butoxide (3 equivalents) | >99% | 95% |
| Lithium isopropoxide (3 equivalents) | 4% | 4% |
| Lithium ethoxide (3 equivalents) | 20% | 18% |
| Lithium methoxide (3 equivalents) | 7% | 6% |

### Example 4a: Comparison of amount of base used in reaction to form trans-rac-1-(2,2-dichloro-3-(diethoxymethyl)cyclopropyl)-3,5-bis(trifluoromethyl)benzene from (E)-1-(3,3-diethoxyprop-1-en-1-yl)-3,5-bis(trifluoromethyl)benzene

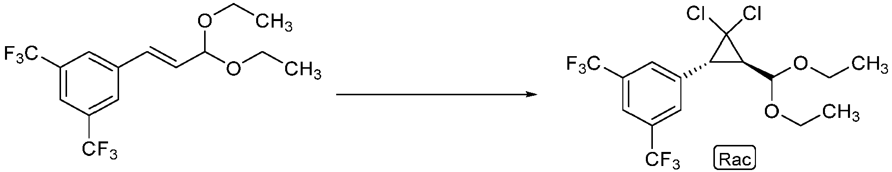

In a vial, (*E*)-1-(3,3-diethoxyprop-1-en-1-yl)-3,5-bis(trifluoromethyl)benzene (200 mg, 0.584 mmol) was dissolved in chloroform (1.9 mL, 23.37 mmol). Lithium *tert*-butoxide (5 to 15 equivalents) was added to the solution in one portion. The mixture was heated to 40 °C and stirred vigorously for 24 hours. After cooling to ambient temperature, the reaction mixture was washed with water (2 mL) twice. The organic layer was separated, dried over anhydrous sodium sulfate, filtered, and concentrated under vacuum by rotary evaporator. The resulting oil was analyzed by ¹H NMR spectroscopy to obtain ¹H NMR yield using 1,4-dinitrobenzene as an internal standard. The results are reported in the table below.

| **TABLE 4a** | | |
|---|---|---|
| **Base (molar equivalents with respect to starting material)** | **Starting Material Conversion** | **Product ¹H-NMR yield** |
| Lithium *tert*-butoxide (5 equivalents) | 76% | 69% |
| Lithium *tert*-butoxide (6 equivalents) | 76% | Not determined |
| Lithium *tert*-butoxide (7 equivalents) | 78% | Not determined |
| Lithium *tert*-butoxide (8 equivalents) | 80% | Not determined |
| Lithium *tert*-butoxide (10 equivalents) | 85% | 84% |
| Lithium *tert*-butoxide (15 equivalents) | 85% | 77% |

### Example 4b: Comparison of amount of base used in reaction to form trans-rac-1-(2,2-dichloro-3-(diethoxymethyl)cyclopropyl)-3,5-bis(trifluoromethyl)benzene from (E)-1-(3,3-diethoxyprop-1-en-1-yl)-3,5-bis(trifluoromethyl)benzene

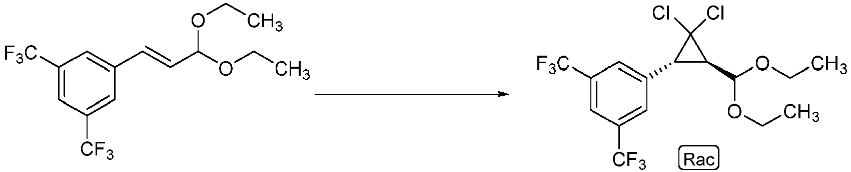

Into a scintillation vial inside the glovebox, lithium *tert*-butoxide was weighed. In a separate vial, (E)-1-(3,3-diethoxyprop-1-en-1-yl)-3,5-bis(trifluoromethyl)benzene (0.20 g, 0.584 mmol, 1 equivalent) and hexachloroacetone (0.445 mL, 2.92 mmol, 5 equivalents) were mixed and then added to the solid base via syringe at 23 °C. The mixture was stirred for 24 hours at 23 °C. 1,3,5-Trimethoxybenzene was added to the reaction mixture and used as internal standard to calculate yield by ¹H NMR spectroscopy. The results are reported in the table below.

| **TABLE 4b** | | |
|---|---|---|
| **Base (molar equivalents with respect to starting material)** | **Starting Material Conversion** | **Product ¹H-NMR yield** |
| Lithium *tert*-butoxide (1 equivalent) | 33% | 32% |
| Lithium *tert*-butoxide (2 equivalents) | 75% | 79% |
| Lithium *tert*-butoxide (3 equivalents) | >99% | 95% |

### Example 5a: Comparison of bases and temperatures used in reaction to form trans-rac-1-(2,2-dichloro-3-(diethoxymethyl)cyclopropyl)-3,5-bis(trifluoromethyl)benzene from (E)-1-(3,3-diethoxyprop-1-en-1-yl)-3,5-bis(trifluoromethyl)benzene

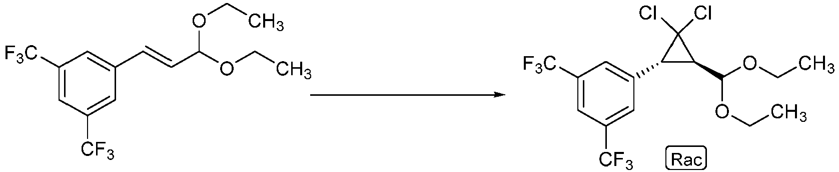

In a vial (*E*)-1-(3,3-diethoxyprop-1-en-1-yl)-3,5-bis(trifluoromethyl)benzene (0.25 g, 0.730 mmol) was dissolved in chloroform (2.356 mL, 29.2 mmol). The mixture was set to the indicated temperature, and a base (6-10 equivalents) was added. The mixture was stirred for 18 hours. Conversion was analyzed by¹H NMR spectroscopy.

| **TABLE 5a** | | | |
|---|---|---|---|
| **Number** | **Base (molar equivalents with respect to starting material)** | **Temperature** | **Conversion to Product** |
| **1** | Potassium *tert*-butoxide (6 equivalents) | 0 °C to 23 °C | Not detected |
| **2** | Sodium *tert*-butoxide (10 equivalents) | 23 °C | Not detected |
| **3** | Magnesium di-*tert*-butoxide (10 equivalents) | 40 °C | Not detected |
| **4** | Lithium bis(trimethylsilyl)amide (6 equivalents) | 23 °C | 5% |
| **5** | Lithium hydroxide monohydrate (6 equivalents) | 23 °C | Not detected |
| Please use caution: in #1, 2, and 4 a vigorous exotherm was observed. | | | |

### Example 5b: Comparison of bases used in reaction to form trans-rac-1-(2,2-dichloro-3-(diethoxymethyl)cyclopropyl)-3,5-bis(trifluoromethyl)benzene from (E)-1-(3,3-diethoxyprop-1-en-1-yl)-3,5-bis(trifluoromethyl)benzene

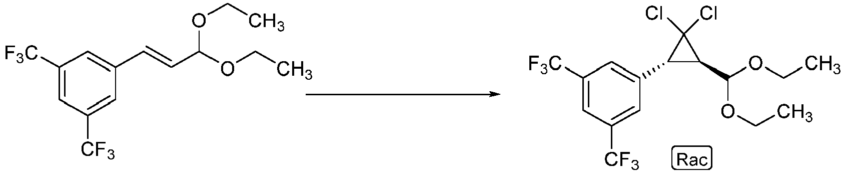

In a vial (*E*)-1-(3,3-diethoxyprop-1-en-1-yl)-3,5-bis(trifluoromethyl)benzene (0.20 g, 0.584 mmol) was dissolved in hexachloroacetone (0.356 mL, 2.337 mmol, 4 equivalents) at 23 °C. Base (1.5-3 equivalents) was added. The mixture was stirred for 24 hours. Conversion was analyzed by ¹H NMR spectroscopy.

| **TABLE 5b** | | |
|---|---|---|
| **Number** | **Base (molar equivalents with respect to starting material)** | **Conversion to Product** |
| **1** | Potassium *tert*-butoxide (1.5 equivalents) | Not detected |
| **2** | Sodium *tert*-butoxide (3 equivalents) | 6% |
| **3** | Magnesium *tert*-butoxide (1.5 equivalents) | Not detected |
| Please use caution: in #1 and 2 a vigorous exotherm was observed. | | |

### Example 6a: Comparison of co-solvents and bases used in reaction to form trans-rac-1-(2,2-dichloro-3-(diethoxymethyl)cyclopropyl)-3,5-bis(trifluoromethyl)benzene from (E)-1-(3,3-diethoxyprop-1-en-1-yl)-3,5-bis(trifluoromethyl)benzene

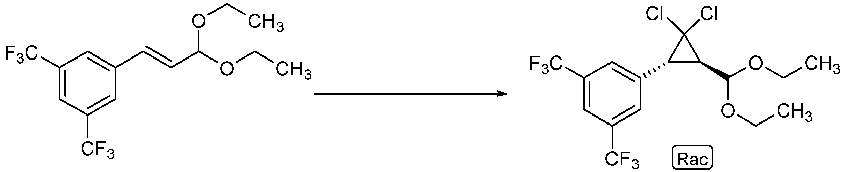

In a vial (*E*)-1-(3,3-diethoxyprop-1-en-1-yl)-3,5-bis(trifluoromethyl)benzene (0.25 g, 0.730 mmol) was dissolved in chloroform (2.356 mL, 29.2 mmol). The mixture was set to the indicated temperature, and a base (6-10 equivalents) was added either as a solid or as a suspension with indicated co-solvents. The mixture was stirred for 18 hours. Conversion was analyzed by ¹H NMR spectroscopy.

| **TABLE 6a** | | | | |
|---|---|---|---|---|
| **Number** | **Co-solvent** | **Base (molar equivalents with respect to starting material)** | **Temperature** | **Conversion to Product** |
| **1** | Hexane (1 Molar relative to base) | Lithium *tert*-butoxide (10 equivalents) | 0 °C to 23 °C | 15% |
| **2** | Toluene (1 Molar relative to base) | Lithium *tert*-butoxide (10 equivalents) | 0 °C to 23 °C | 28% |
| **3** | Diglyme (1 Molar relative to base) | Lithium *tert*-butoxide (10 equivalents) | 0 °C to 23 °C | 13% |
| 4 | Tetrahydrofuran (THF) (1 Molar relative to base) | Lithium *tert*-butoxide (10 equivalents) | 0 °C to 23 °C | 17% |
| **5** | Toluene (1 Molar relative to base) | Lithium bis(trimethylsilyl)amide (6 equivalents) | 0 °C to 23 °C | 4% |
| **6** | Toluene (5.6 Molar relative to base) | Lithium bis(trimethylsilyl)amide (6 equivalents) | 0 °C to 23 °C | 44% |
| Please use caution: in #5 and 6 a vigorous exotherm was observed. | | | | |

### Example 6b: Comparison of co-solvents used in reaction to form trans-rac-1-(2,2-dichloro-3-(diethoxymethyl)cyclopropyl)-3,5-bis(trifluoromethyl)benzene from (E)-1-(3,3-diethoxyprop-1-en-1-yl)-3,5-bis(trifluoromethyl)benzene

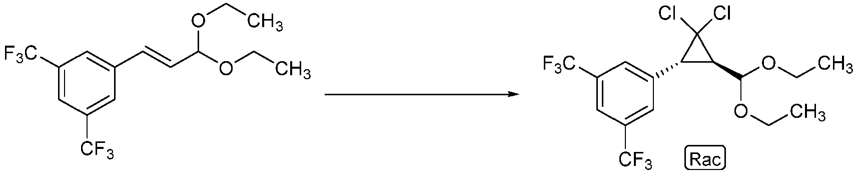

In a vial (*E*)-1-(3,3-diethoxyprop-1-en-1-yl)-3,5-bis(trifluoromethyl)benzene (0.20 g, 0.584 mmol) was dissolved in hexachloroacetone (0.445 mL, 2.92 mmol, 5 equivalents) and the indicated co-solvent. The mixture was set to the indicated temperature, and lithium tert-butoxide (0.234 g, 2.92 mmol) was added. The mixture was stirred for 18 hours. Conversion was analyzed by ¹H NMR spectroscopy.

| TABLE 6b | | |
|---|---|---|
| Co-solvent | Temperature | Conversion to Product |
| Chloroform (1.2 Molar relative to starting material, and less than the molar amount of hexachloroacetone) | 23 °C | >99% |
| Dimethyl carbonate (1.2 Molar relative to starting material) | 23 °C | 20% |

### Example 7: Synthesis of (5R,6R)-5,6-bis((1R,3R)-3-(3,5-bis(trifluoromethyl)phenyl)-2,2-dichlorocyclopropyl)-2,2,3,3,8,8,9,9-octamethyl-4,7-dioxa-3,8-disiladecane

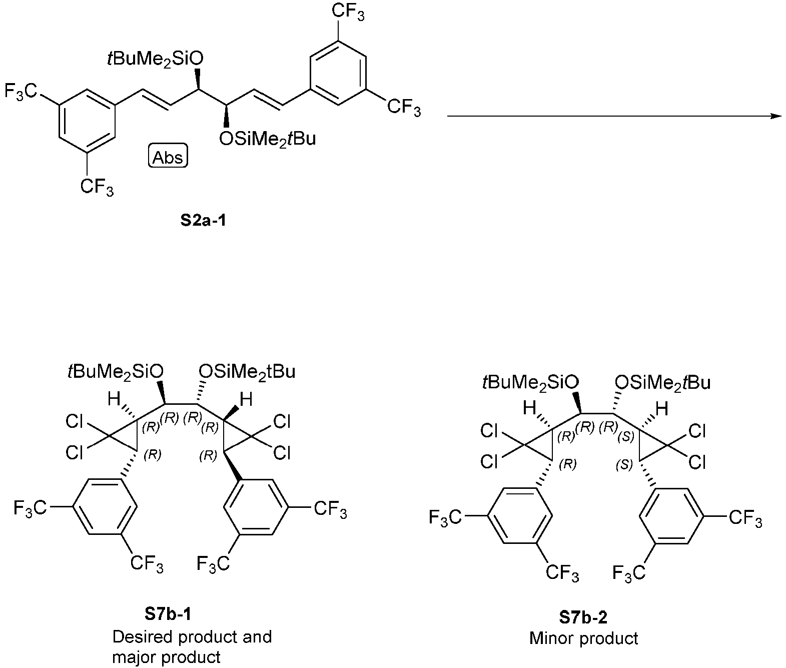

In a vial (5*R,*6*R*)-5,6-bis((*E*)-3,5-bis(trifluoromethyl)styryl)-2,2,3,3,8,8,9,9-octamethyl-4,7-dioxa-3,8-disiladecane (0.25 g, 0.326 mmol) was mixed with hexachloroacetone (0.495 mL, 3.26 mmol). Lithium 2-methylpropan-2-olate (0.130 g, 1.630 mmol) was added in one portion. The mixture was stirred overnight at ambient temperature. After 18 hours, analysis by ¹H NMR spectroscopy showed >99% conversion of the starting material to the desired product with a diastereoselectivity ratio (dr) of ~5.5:1 favoring desired product. The reaction was quenched with water and the organic layer was separated, dried, and concentrated to an oil. No isolated yield was obtained.

### Example 8: Synthesis of (5R,6R)-5,6-bis((1R,3R)-3-(3,5-bis(trifluoromethyl)phenyl)-2,2-dichlorocyclopropyl)-2,2,3,3,8,8,9,9-octamethyl-4,7-dioxa-3,8-disiladecane

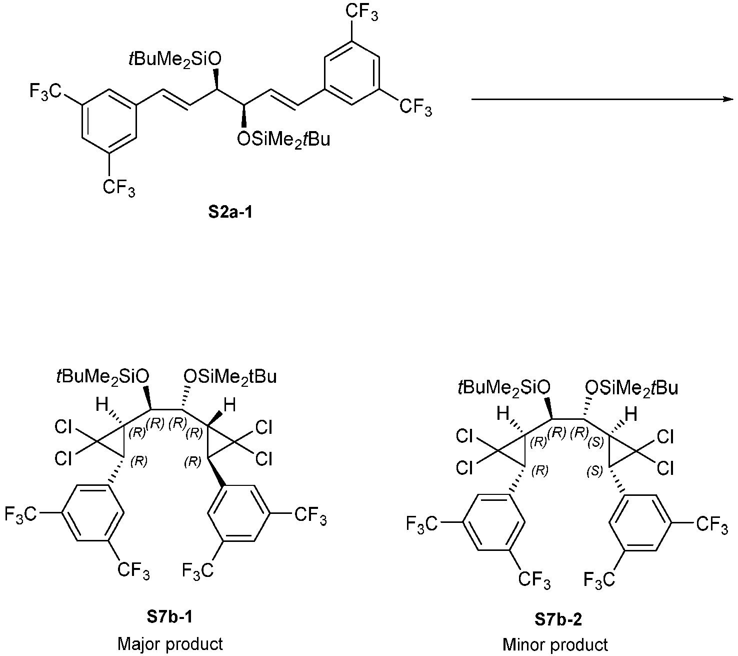

In a vial (5*R*,6*R*)-5,6-bis((*E)*-3,5-bis(trifluoromethyl)styryl)-2,2,3,3,8,8,9,9-octamethyl-4,7-dioxa-3,8-disiladecane (0.25 g, 0.326 mmol) and lithium 2-methylpropan-2-olate (0.391 g, 4.89 mmol) were added. To the solids, chloroform (1.841 mL, 22.82 mmol) was added. The mixture was heated to 40 °C and stirred for 18 hours affording desired product with a significant amount of mono-cyclopropanated material. An additional 5 equivalents of lithium 2-methylpropan-2-olate were added (0.13 g) and the mixture was stirred for an additional day. After 40 hours, ¹H NMR analysis showed ~15% of alkene mono-cyclopropanated species and -85% conversion to desired product (dr was not determined). The reaction was quenched with water and the organic layer was separated, dried, and concentrated to an oil. No isolated yield was obtained.

### Example M1: Synthesis of (5R,6R)-2,2,3,3,8,8,9,9-octamethyl-5,6-divinyl-4,7-dioxa-3,8-disiladecane

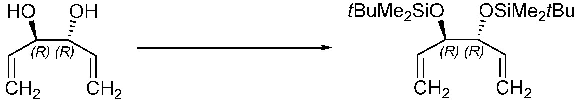

In a four-neck reactor equipped with a mechanical stirrer, condenser, nitrogen inlet and temperature probe, (3R,4R)-hexa-1,5-diene-3,4-diol (60 g, 526 mmol) was stirred in anhydrous N,N-dimethylformamide (DMF, 394 mL) (It should be noted that DMF could have been replaced by acetonitrile, or a mixture of acetonitrile and DMF could have been used). The mixture was cooled to 0-5 °C. Imidazole (140 g, 2050 mmol) was added. A solution of tert-butylchlorodimethylsilane (158 g, 1051 mmol, 2 equivalents) in heptane (131 mL) was added via addition funnel (temperature < 5 °C). The mixture was warmed to ambient temperature and stirred for 20 hours. Methanol (15.95 mL, 394 mmol, 0.75 equivalents) was added with stirring at ambient temperature. After 1 hour, the mixture was charged with heptane (1.5 L) and was stirred vigorously for 10-15 minutes. Stirring was stopped and the layers separated. The heptane portion (top layer) was collected and washed/partitioned with acetonitrile (2 x 100 mL). The heptane portion was concentrated to provide the title compound as a colorless oil, which was used without further manipulation (178 g, 95% yield, 96% purity): ¹H NMR (400 MHz, CDCl₃) δ 6.28 - 5.60 (m, 2H), 5.26 - 5.04 (m, 4H), 4.17 - 4.06 (m, 2H), 0.91 (s, 18H), 0.07 (s, 6H). 0.05 (s, 6H).

### Example M2: Synthesis of (5R,6R)-5,6-bis((E)-3,5-bis(trifluoromethyl)styryl)-2,2,3,3,8,8,9,9-octamethyl-4,7-dioxa-3,8-disiladecane

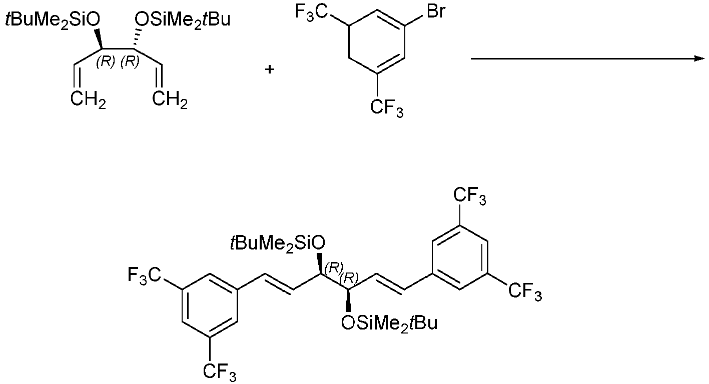

The reaction was conducted under nitrogen in a four-neck 1-L jacketed reactor equipped with a mechanical stirrer, temperature probe, nitrogen inlet and baffle. 1-Bromo-3,5-bis(trifluoromethyl)benzene (115 g, 394 mmol) and (5R,6R)-2,2,3,3,8,8,9,9-octamethyl-5,6-divinyl-4,7-dioxa-3,8-disiladecane (50 g, 146 mmol) were stirred in DMF (365 mL). Potassium carbonate (38.3 g, 277 mmol), sodium acetate (15.56 g, 190 mmol) and diacetoxypalladium (1.31 g, 5.84 mmol, 4 mol %) were added. The mixture was heated to an internal temperature of 66 °C. After 24 hours, heating was ceased. While the reaction was warm, acetonitrile (500 mL) and heptane (1 L) were added with stirring. Upon cooling to ambient temperature stirring was stopped and the layers separated. The DMF portion (bottom layer) along with solid salts were drained from the reactor. The heptane portion (top layer) was collected. The bottom layer was back extracted with heptane (500 mL). The heptane layers were combined, washed with acetonitrile (2 x 200 mL), and concentrated to provide the title compound as an off-white solid (99.3 g, 84% yield): ¹H NMR (400 MHz, CDCl₃) δ 7.70 (s, 6H), 6.62 (dd, *J* = 16.0, 1.1 Hz, 2H), 6.43 (ddd, *J* = 16.0, 3.3, 1.2 Hz, 2H), 4.66 - 4.21 (m, 2H), 0.98 (s, 18H), 0.15 (s, 6H), 0.12 (s, 6H); ¹⁹F NMR (376 MHz, CDCl₃) δ -63.09.

## Claims

1. A process comprising halocyclopropanating **S1a** to produce **S1b** in the presence of hexachloroacetone and lithium *tert*-butoxide 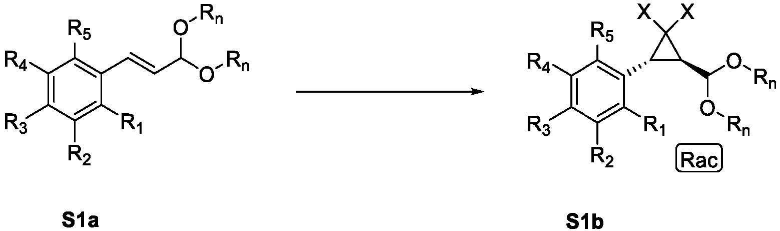 wherein
**(a) R₁, R₂, R₃, R₄,** and **R₅** are each independently H, F, Cl, Br, I, CN, NH₂, NO₂, (C₁-C₄)alkyl, (C₁-C₄)alkoxy, (C₁-C₄)haloalkyl, or (C₁-C₄)haloalkoxy;
**(b)** each **Rₙ** is independently a (C₁-C₄)alkyl, or both **Rₙ** substituents together form a (C₂-C₆)alkyl link between the two oxygen atoms; and
**(c)** each**X** is Cl.

2. A process comprising halocyclopropanating **S2a** to produce **S2b-major product** in the presence of hexachloroacetone and lithium *tert*-butoxide 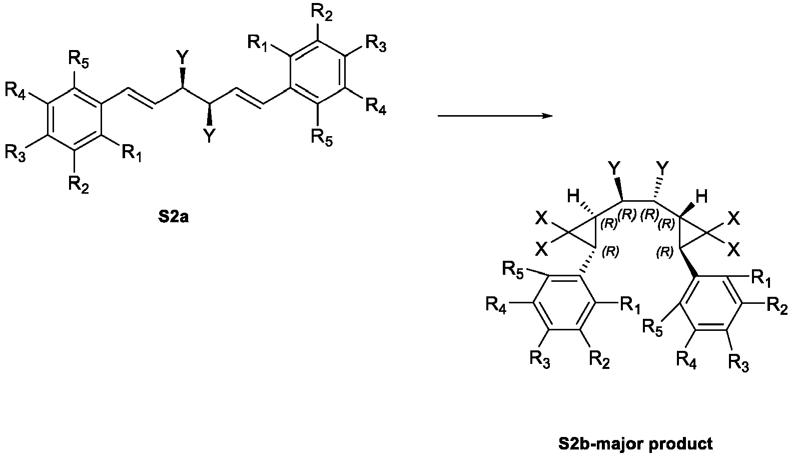 wherein
**(a) R₁, R₂, R₃, R₄,** and **R₅** are each independently H, F, Cl, Br, I, CN, NH₂, NO₂, (C₁-C₄)alkyl, (C₁-C₄)alkoxy, (C₁-C₄)haloalkyl, or (C₁-C₄)haloalkoxy;
**(b)** each **Y** is independently a OH, OSi((C₁-C₄)alkyl)₃, OSi((C₁-C₄)alkyl)₂((C₁-C₄)alkyl-aryl), OSi((C₁-C₄)alkyl)₂(aryl), OSi((C₁-C₄)alkyl)(aryl)₂, OC(O)(C₁-C₄)alkyl, OC(O)O(C₁-C₄)alkyl, OC(O)NH(C₁-C₄)alkyl, O(C₁-C₄)alkyl-aryl, O-aryl, tetrahydropyranyl, or 1,3-dioxolanyl; and
**(c)** each**X** is Cl.

3. A molecule having the following structure

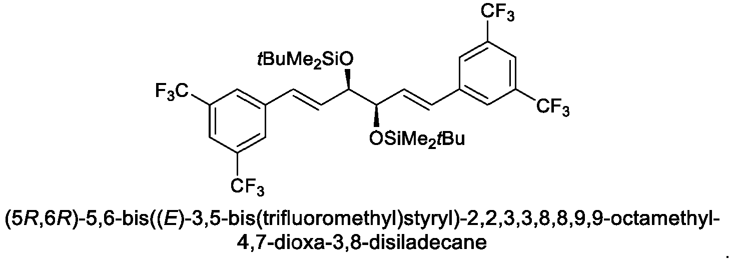

4. A molecule having the following structure

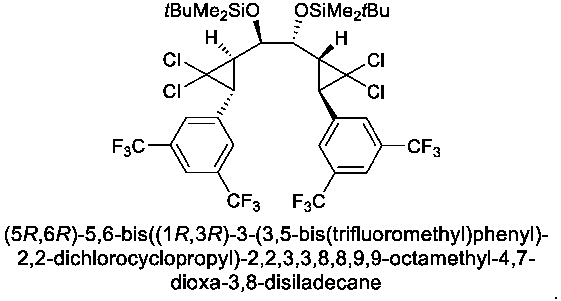

## Patentansprüche

1. Verfahren, umfassend das Halogencyclopropanieren von S1a zu S1b in Gegenwart von Hexachloraceton und Lithium-tert.-butoxid 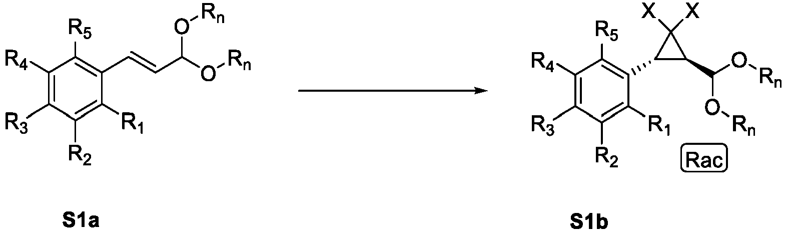 wobei
(a) R₁, R₂, R₃, R₄ und R₅ jeweils unabhängig für H, F, Cl, Br, I, CN, NH₂, NO₂, (C₁-C₄) -Alkyl, (C₁-C₄)-Alkoxy, (C₁-C₄)-Halogenalkyl oder (C₁-C₄)-Halogenalkoxy stehen;
(b) Rₙ jeweils unabhängig für ein (C₁-C₄)-Alkyl steht oder beide Rₙ-Substituenten zusammen eine (C₂-C₆)-Alkyl-Verknüpfung zwischen den beiden Sauerstoffatomen bilden; und
(c) X jeweils für Cl steht.

2. Verfahren, umfassend das Halogencyclopropanieren von S2a zu S2b-Hauptprodukt in Gegenwart von Hexachloraceton und Lithium-tert.-butoxid 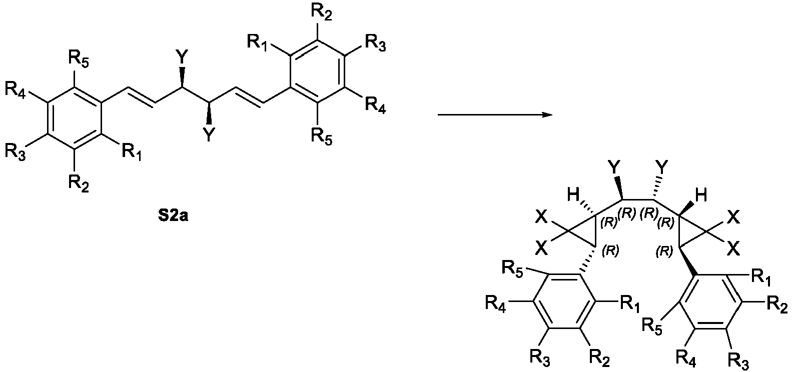 wobei
(a) R₁, R₂, R₃, R₄ und R₅ jeweils unabhängig für H, F, Cl, Br, I, CN, NH₂, NO₂, (C₁-C₄)-Alkyl, (C₁-C₄)-Alkoxy, (C₁-C₄)-Halogenalkyl oder (C₁-C₄)-Halogenalkoxy stehen;
(b) Y jeweils unabhängig für OH, OSi((C₁-C₄)-Alkyl)₃, OSi ((C₁-C₄)-Alkyl)₂((C₁-C₄)-alkyl-aryl), OSi((C₁-C₄)-Alkyl)₂(aryl), OSi((C₁-C₄)-Alkyl) (aryl)₂, OC(O)(C₁-C₄)-Alkyl, OC(O)O(C₁-C₄)-Alkyl, OC(O)NH(C₁-C₄)-Alkyl, O(C₁-C₄)-Alkyl-aryl, O-Aryl, Tetrahydropyranyl oder 1,3-Dioxolanyl steht; und
(c) X jeweils für Cl steht.

3. Molekül mit der folgenden Struktur: 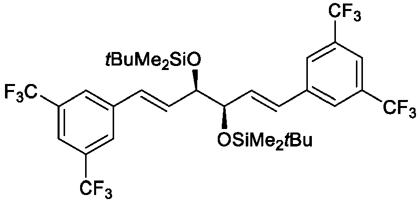 (5R,6R)-5,6-Bis((E)-3,5-bis(trifluormethyl)styryl)-2,2,3,3,8,8,9,9-octamethyl-4,7-dioxa-3,8-disiladecan.

4. Molekül mit der folgenden Struktur: 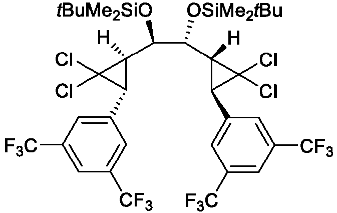 (5R,6R)-5,6-Bis((1R,3R)-3-(3,5-bis(trifluormethyl)phenyl)-2,2-dichlorocyclopropyl)-2,2,3,3,8,8,9,9-octamethyl-4,7-dioxa-3,8-disiladecan.

## Revendications

1. Procédé comprenant l'halogénocyclopropanation de S1a pour produire S1b en présence d'hexachloroacétone et de tert-butoxyde de lithium 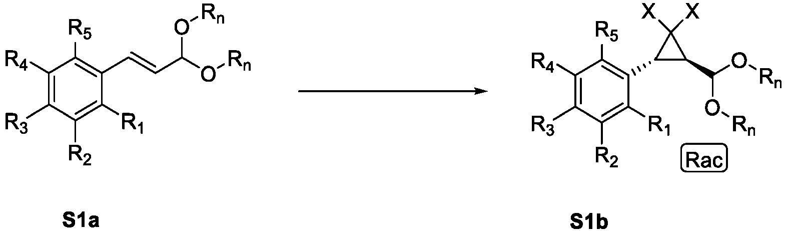 dans lesquelles
(a) R₁, R₂, R₃, R₄, et R₅ sont chacun indépendamment H, F, Cl, Br, I, CN, NH₂, NO₂, (C₁-C₄)alkyle, (C₁-C₄)alcoxy, (C₁-C₄)halogénoalkyle, ou (C₁-C₄)halogénoalcoxy ;
(b) chaque Rₙ est indépendamment un (C₁-C₄)alkyle, ou les deux substituants Rₙ forment ensemble un lien (C₂-C₆)alkyle entre les deux atomes d'oxygène ; et
(c) chaque X est Cl.

2. Procédé comprenant l'halogénocyclopropanation de S2a pour produire un produit majoritaire S2b en présence d'hexachloroacétone et de tert-butoxyde de lithium 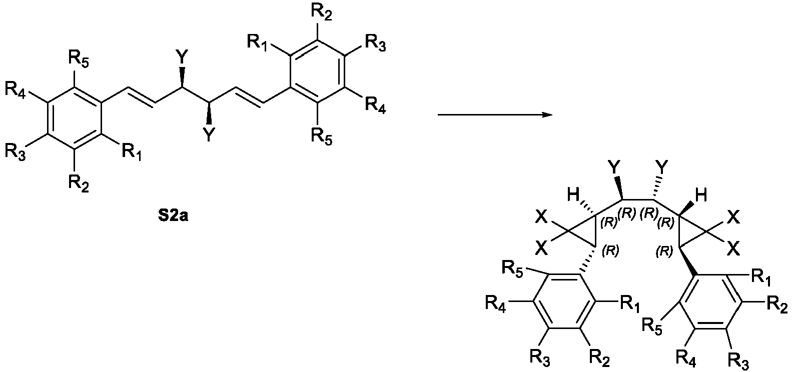 dans lesquelles
(a) R₁, R₂, R₃, R₄, et R₅ sont chacun indépendamment H, F, Cl, Br, I, CN, NH₂, NO₂, (C₁-C₄)alkyle, (C₁-C₄)alcoxy, (C₁-C₄)halogénoalkyle, ou (C₁-C₄)halogénoalcoxy ;
(b) chaque Y est indépendamment un OH, OSi((C₁-C₄)alkyle)₃, OSi((C₁-C₄)alkyle)₂((C₁-C₄)alkyl-aryle), OSi((C₁-C₄)alkyle) ₂(aryle), OSi((C₁-C₄)alkyle)(aryle) ₂, OC(O)(C₁-C₄)alkyle, OC(O)O(C₁-C₄)alkyle, OC(O)NH(C₁-C₄)alkyle, O(C₁-C₄)alkyl-aryle, O-aryle, tétrahydropyranyle, ou 1,3-dioxolanyle ; et
(c) chaque X est Cl.

3. Molécule ayant la structure suivante 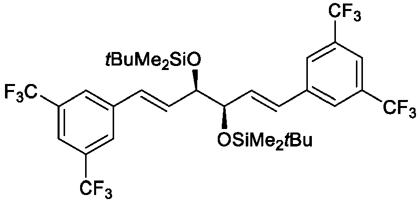 (5R,6R)-5,6-bis((E)-3,5-bis(trifluorométhyl)styryl)-2,2,3,3,8,8,9,9-octaméthyl-4,7-dioxa-3,8-disiladécane.

4. Molécule ayant la structure suivante 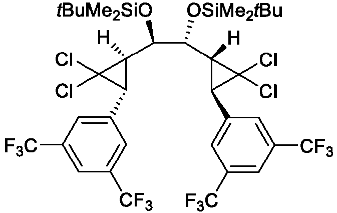 (5R,6R)-5,6-bis((lR,3R)-3-(3,5-bis(trifluorométhyl)phényl)-2,2-dichlorocyclopropyl)-2,2,3,3,8,8,9,9-octaméthyl-4,7-dioxa-3,8-disiladécane.
